# EUROPEAN PATENT SPECIFICATION

(11) **EP 4 157 037 B1**
(45) Date of publication and mention of the grant of the patent: **23.07.2025**
(21) Application number: 21726658.4
(22) Date of filing: 20.05.2021
(51) Int. Cl.: A46B 15/00, A61N 1/05, A46B 9/04, A61C 17/22

(54) **CLEANING UNIT FOR AN ORAL CLEANING AND/OR TREATMENT DEVICE**
REINIGUNGSEINHEIT FÜR EINE VORRICHTUNG ZUR ORALEN REINIGUNG UND/ODER BEHANDLUNG
UNITÉ DE NETTOYAGE POUR UN DISPOSITIF DE NETTOYAGE ET/OU DE TRAITEMENT BUCCO-DENTAIRE

(30) Priority: 28.05.2020 EP 20176962
(43) Date of publication of application: 05.04.2023
(73) Proprietor: Koninklijke Philips N.V., 5656 AG Eindhoven (NL)
(72) Inventor: SCHEFFERS, Lucas, Petrus, Henricus, 5656 AE Eindhoven (NL); BHAT, Ravindra, 5656 AE Eindhoven (NL); RONDA, Cornelis, Reinder, 5656 AE Eindhoven (NL); GERHARDT, Lutz, Christian, 5656 AE Eindhoven (NL); JOHNSON, Mark, Thomas, 5656 AE Eindhoven (NL)
(74) Representative: Philips Intellectual Property & Standards
(86) International application number: PCT/EP2021/063399
(87) International publication number: WO 2021/239549

(56) References cited:
- WO-A1-2017/216606
- WO-A1-2019/016286
- JP-A- 2007 167 527
- US-A1- 2012 233 790
- US-A1- 2016 278 897
- US-B2- 10 201 701
- US-B2- 7 886 398

## Description

### FIELD OF THE INVENTION

The present invention relates to a cleaning unit for an RF-energy based oral cleaning and/or treatment device, i.e. an oral cleaning and/or treatment device employing electromagnetic energy for a cleaning function.

### BACKGROUND OF THE INVENTION

Radio-frequency electromagnetic emissions can be used to provide a cleaning or treatment function in the oral cavity. In particular, an oral cleaning or treatment device can include a cleaning or treatment unit, such as a head portion, for insertion into an oral cavity of a user, which cleaning unit portion includes two or more electrodes coupled to an RF signal generator. The signal generator drives the electrodes with an RF signal which causes an RF field to be generated around and between the electrodes.

According to US10201701B2, when the RF field interacts with surfaces of the teeth and gums, it provides a cleaning function by loosening the bonds between impurities and the surfaces in the mouth. In particular, RF fields generated in this way can remove dental plaque, and also dental calculus. Staining of teeth can also be reduced.

US10201701B2 describes an electric toothbrush comprising an RF generator, two RF electrodes, and a dielectric barrier situated between the two RF electrodes in the form of a silicone strip. The dielectric barrier has a height which extends up to the level of the distal tips of the brush bristles. The barrier forces RF waves transmitted between the electrodes to travel over the top of the barrier, thus reaching the area where the bristles engage with the surfaces of the teeth and gums in use.

The distal tips of the brush bristles are rubbed against the surfaces of teeth in the usual manner to clean the teeth, while at the same time, the RF electrodes emit RF waves which provide a cleaning function at the same surface which is being brushed, at the level of the tips of the bristles.

Developments in the field of RF oral cleaning and/or treatment devices are generally sought.

US 2016/278897 A1 discloses a tooth brushing system comprising a flexible tray comprising one or more vibratory transducers removably coupled to one or more brushing panels.

US 2012/233790 A1 discloses a brush comprising bristle bundles embedded in the brush head, A bristle contained in the bristle bundles includes a core which consists of a conductive member, and a sheath which consists of an insulating member and covers the core. The core is exposed at the tip portion of the bristle and functions as an electrode for passing current into the mouth.

US 7886398 B2 discloses an oral cavity cleaning tool for the purpose of sterilization of the inside of oral cavity which carries out sterilization by using effective chlorine that is obtained through electrolysis.

JP 2007/167527 A discloses an artificial tooth cleaner which includes a pair of electrodes and a power source to generate potential differences between the pair of electrodes. Effective chlorine is produced in a range of 5 ppm to 35 ppm if current flows through the pair of electrodes while kept in contact with solution containing chloride ions.

WO 2017/216606 A1 discloses an oral cleaning device including an RF generator connected to electrodes located on a head portion, and a non-conductive barrier located on the head portion that separates the electrodes from each other.

### SUMMARY OF THE INVENTION

The invention is defined by the independent claims. The dependent claims define advantageous embodiments.

According to examples in accordance with an aspect of the invention, there is provided a cleaning unit for an oral cleaning and/or treatment device, the cleaning unit for being received in an oral cavity of a user, the cleaning unit comprising:
a first protruding structure, extending outward from a surface of the cleaning unit, the first protruding structure comprising one or more first electrodes,
a second protruding structure extending outward from a surface of the cleaning unit and comprising one or more second electrodes,
the second protruding structure spaced from the first protruding structure by a gap,
wherein the gap is arranged such that at least one tooth is receivable in the gap between the first and second protruding structures, with the first and second protruding structures extending either side of the gap (and therefore the tooth), and wherein, with the at least one tooth received in the gap, the first and second protruding structures extend at least partway down outer side surfaces of the tooth.

Embodiments of the invention are based on the concept of providing the electrodes in a form such a tooth can be fitted in-between the two sets of electrodes, with the electrodes held positioned at least partway down the outer side faces of the tooth. This way the tooth itself acts as the insulating barrier between the electrodes, replacing the need for a dedicated barrier element. Furthermore, this arrangement improves cleaning of interproximal spaces between teeth because the electrodes are positioned closer to the spaces, and at the same height level as the spaces. Because the tooth provides an insulating barrier, the RF energy is naturally induced to flow between the electrodes via the interproximal spaces, i.e. along a pathway which extends through the interproximal spaces at either side of the tooth.

Thus, embodiments of the present invention provide an enhanced cleaning capability compared to prior art devices.

It is noted that although a cleaning unit is referred to above, the device may additionally or alternatively be a treatment unit for treating one or more areas in the oral cavity with RF energy. Thus, reference in this disclosure to a cleaning unit may be understood as reference to a cleaning and/or treatment unit.

The gap preferably extends from the first protruding structure to the second protruding structure. The gap may consist of a space extending from the first protruding structure to the second protruding structure. The gap may be an open space extending (uninterrupted) from the first protruding structure to the second protruding structure. A space for example means an air gap.

The gap is for receiving the full width of a tooth between the first and second protruding structures. The gap is of a size suitable for receiving the full width of an average molar tooth of an average adult human.

The protruding structures may be arranged such that, with the tooth received in the gap, the protruding structures lie directly adjacent to side surfaces of the tooth (i.e. facing the tooth surfaces, e.g. with nothing further between the protruding structure and the tooth surface). The protruding structures may make contact with side surfaces of the tooth.

The protruding structures may be arranged such that, with the tooth received in the gap, the protruding structures are positioned adjacent to, and either side of, an interdental space next to the tooth.

The cleaning unit is a unit for an RF-energy based oral cleaning or treatment device. For example, each of the first and second electrodes may be suitable for being driven with an RF drive signal to generate an RF electromagnetic field.

The cleaning unit is for being received within the oral cavity of a user for performing an oral cleaning and/or treatment function.

The cleaning unit may be for example a head for an oral cleaning and/or treatment device such as a toothbrush or other device, or may be a different form of cleaning or treatment unit, such a mouthpiece unit for fitting into the mouth.

The first and second protruding structures may each comprise a set of one or more protruding members.

Each protruding structure may thus be comprised of a single protruding member for instance, or a plurality of protruding members.

Each protruding structure may in some examples comprise a single laminar protruding member which defines an elongate footprint on the cleaning unit surface, i.e. it extends from a line on the cleaning unit surface.

Each protruding structure may alternatively comprise a plurality of protruding members. Each protruding member may comprise a respective electrode, or just a subset of the protruding members may comprise an electrode.

For example, in one set of embodiments (described further below), each protruding member may form a respective bristle, and each protruding structure may comprise a respective tuft or bundle of bristles. However, this represents just one example arrangement.

At least a portion of the one or more protruding members of each protruding structure may be flexible. This can be useful for permitting the tooth to be accommodated in the gap between the first and second protruding structures.

For example, at least a portion of the protruding member(s) of the first and second protruding structures may be adapted to flex to receive the tooth in the gap. The protruding members may be such that, when the tooth is received in the gap, the members resiliently engage with outer faces of the tooth.

The advantage of forming the protruding structures of multiple protruding members (e.g. a bundle or tuft of protruding members) is that the protruding structures can better conform to the shape of the tooth accommodated in the gap, or more easily deform outwards to accommodate the tooth if necessary.

According to some embodiments, the cleaning unit may further comprise a further protruding structure extending outward from a surface of the cleaning unit, the further protruding structure comprising one or more further electrodes, and the further protruding structure disposed in the gap between the first and second protruding structures, and protruding to a shorter height the first and second protruding structures.

The further protruding structure comprises a shorter set of one or more electrodes which allow an RF field to be generated in the vicinity of the crown of the tooth when the tooth is received in the gap. At the same time, the first and second protruding structures can be driven to generate an RF field to treat the sides of the tooth, and particularly the interproximal spaces between the teeth.

There are different options as to how the protruding structures may be implemented, which may in part depend upon the intended application of the cleaning unit, i.e. the type of oral care device that the cleaning unit will be used for.

For example, according to one set of embodiments, the cleaning unit may comprise a platen, and further comprise a plurality of bristles extending outwardly from a first face of the platen for cleaning teeth, and wherein the plurality of bristles are shaped and/or arranged to permit entry of a tooth into said gap.

The tooth in this example may be received into the gap in a direction down toward the platen first surface, with the first and second protruding structures being lowered down over the tooth in question with their distal tips pointing in the direction of movement.

According to this set of embodiments, the cleaning unit may be a head for a toothbrush device for example.

For example, the bristles may be shaped, profiled, or simply positioned in such a way as to not impede receipt of the tooth from above the platen down into the gap between the first and second protruding structures. The configuration of the bristles is so as to permit entry and accommodation of a tooth within said gap.

For example, distal ends of the plurality of bristles may define a profile shaped to permit entry and accommodation of a tooth within said gap.

The first and second protruding structures may be at least partially formed by first and second subsets respectively of said plurality of bristles.

One or more of the bristles of the first subset of bristles may comprise the one or more first electrodes, and one or more bristles of the second subset may comprise the one or more second electrodes.

Each of the first electrodes and second electrodes may be in the form of bristles. For example, one or more of the bristles may be formed of a conductive polymer, or otherwise configured to act as an electrode. Alternatively, one or more of the bristles may carry or integrate the electrodes on or within it.

In some examples, the first subset of bristles may be arranged in a first spatial group and the second subset of bristles may be arranged in a second spatial group, the first and second spatial groups having a spacing between them defining said gap.

The spatial groups may each define an elongate footprint on the surface of the cleaning unit, such that they each define a linear or elongate profile.

In some examples, the cleaning unit may comprise an intermediate set of bristles, positioned between the first subset of bristles and the second subset of bristles, and the intermediate set of bristles having a shorter length than each of the first and second subsets of bristles, and wherein distal ends of the intermediate set of bristles define a lower boundary to said gap for receiving the tooth.

The intermediate set of bristles may form a further protruding structure, and may comprise one or more further electrodes in some examples. Alternatively, it may simply permit brushing of a crown of the tooth received in the gap at the same time as RF energy is applied by the electrodes of the first and second protruding structures.

According to one or more embodiments, each of the first and second subsets of bristles may comprise a plurality of curved bristles, the curved bristles of each subset arranged to curve inward toward the gap between the first and second protruding structures.

The protruding structures in this set of embodiments thus define a U-shape receiving gap for the tooth. By providing the electrodes within bristle bundles which curve inward toward the gap, when the tooth is received in the gap, the tips of the bristles are biased against side surfaces of the tooth. This both allows the tooth to be brushed by the bristles for a cleaning function, but also ensures that the electrodes among the bristles are held close against the sides of the teeth, maximizing the RF cleaning or treatment functionality.

According to any of the above embodiments, each of the protruding structures may define an elongate footprint on the surface of the cleaning unit from which it protrudes, such that the gap has a length defined by a length of said elongate footprint.

The gap may be elongate.

Above have been discussed a set of examples in which the cleaning unit comprises bristles, for providing a dental brushing function.

**In** other examples, the cleaning unit may take a different form. For example, the cleaning unit may take the form of a non-brushing mouthpiece, or a head for a non-brushing oral treatment device. Brushing mouthpiece devices are also an option.

**In** accordance with one or more embodiments, the cleaning unit may comprise a support body, the support body having a first side facing a first direction, and a second side facing in a second direction, opposite to the first direction, and wherein the first and second protruding structures extend outward from the first side.

The cleaning unit may further comprise a third protruding structure extending outward from a surface of the second side of the support body and comprising one or more third electrodes, and a fourth protruding structure extending outward from the second side of the support body and comprising one or more fourth electrodes, and the third protruding structure spaced from the fourth protruding structure by a further gap for receiving a tooth therein.

This structure allows teeth from the upper and lower sets of teeth to be both received and cleaned at the same time, with one tooth received in the first gap, on the first (upper) side, and a second tooth received in the second gap beneath the first gap, and with the support body providing a spacer or barrier between the teeth.

The third and fourth protruding structures may be arranged in alignment with the first and second protruding structures respectively, such that the gap and the further gap are in alignment along an axis parallel said first and second directions.

The cross-section of the cleaning unit in this example defines an H-shape, with the first and second protruding structures forming upper arms of the H-shape, the third and fourth protruding structures forming lower arms of the H-shape and the support-core forming the cross-bar for the H-shape.

The protruding structures may each define an elongate footprint on the cleaning unit surface, so that the gaps have a length commensurate with the length of these elongate footprints.

In some examples the cleaning unit may be a mouthpiece unit, wherein the support body extends in a U-shaped or horse-shoe shape for extending around the contour of the teeth in the mouth. The protruding structures on each side of the core may follow and extend continuously along the same curved shape of the core to effectively define an upper channel (formed by the gap between the first and second protruding structures) for receiving the upper teeth and a lower channel (formed by the gap between the third and fourth protruding structures) for receiving the lower teeth.

The cleaning unit may be formed by a single integral or unitary body so that the core and the protruding structures are part of the same integral structure. Thus, the cleaning unit may be formed as a one-piece structure. This may for example be flexible in some examples to permit confirming to the contour of the teeth or mouth.

Examples in accordance with a further aspect of the invention provide an oral cleaning and/or treatment device comprising:
a cleaning unit in accordance with any of the examples or embodiments discussed above or described below, or in accordance with any claim of this application; and
an RF generator in electrical communication with the first electrodes and the second electrodes and configured to drive the electrodes with a drive signal (e.g. an alternating potential or current) to induce an RF alternating (electromagnetic) field in an area around and between the electrodes, to provide an oral cleaning function when the cleaning unit is received in an oral cavity.

The RF energy interacts with surfaces of teeth and gums to provide a cleaning function.

According to one or more embodiments, the device may further comprise a sensing function for sensing in a region between the first and second protruding structures, for example for sensing physical bodies or objects brought into the gap between the first and second protruding structures.

The sensing function may be based on monitoring electrical characteristics of an electrical circuit comprising the first and second electrodes. For example, the RF generator may be electrically connected to the first and second electrodes and arranged to apply an alternating drive signal across the electrodes. The circuit comprising the RF generator, and the first and second electrodes forms an RF generation circuit. Sensing may be based on monitoring electrical characteristics of the generation circuit.

The device may be further adapted to perform sensing in a region between the first electrodes and the second electrodes, based on sensing variations in one or more electrical characteristics of a circuit comprising the first and second electrodes.

In particular, the device may comprise a controller. The controller may be adapted to detect presence of a dental feature or structure in a region between the first and second protruding structures (e.g. in the gap between them), based on sensing variations in one or more electrical characteristics of the circuit comprising the first and second electrodes.

In advantageous embodiments, the controller may be adapted to sense presence of a tooth received in the gap between the first and second protruding structure, or sense presence of an interdental space within the gap.

It may sense deflection of one or more of the protruding members caused by insertion or receipt of one or more of the members in an interdental (interproximal) space.

The sensing may be based on sensing variation in a voltage across the first and second electrodes.

The sensing may be based on directly or indirectly sensing a change in capacitance between the first and second electrodes.

For instance, for a first and second protruding structure separated by a gap of distance, D, if the protruding structures flex outward from the gap slightly as a tooth is received in the gap, the distance, D, changes (increases) and the capacitance or impedance between the electrodes changes accordingly. The change in capacitance may be measured based on sensing a voltage change over the electrode pair (formed of the first electrode(s) and second electrode(s)).

Likewise, if one or both of the protruding structures is received or inserted in an interdental space, this may also alter the gap distance D between the first and second electrodes (for instance reduce it), leading to a detectable change in capacitance.

Furthermore, if a tooth is received in the gap, the presence of this object in the space between the electrodes may change the overall electrical permittivity of the space between the electrodes which may change the effective capacitance between them in a detectable way.

The oral cleaning and/or treatment device may include a sensing unit arranged to sense or monitor one or more electrical characteristics of the circuit comprising the first and second electrodes, e.g. the generator circuit. A controller may be coupled to the sensor unit and arranged to receive a sensor signal output from the sensor unit, and adapted to detect receipt or insertion of an object or structure in the gap between the protruding structures based on the sensor signal output.

The controller may be operatively coupled with the RF generator, and adapted to configure operation settings of the RF generator based on the result of the sensing. For example, it may be adapted to change a drive scheme of the drive signal.

The controller may be adapted to trigger one or more response actions responsive to sensing a particular dental feature or structure, such as an interproximal (interdental) space, in the region between the protruding structures.

For example, an intensity or power level of the RF field may be increased (e.g. by increasing an amplitude or frequency of the RF drive signal, responsive to detecting a tooth or interdental space received in the gap.

The controller may be arranged to adjust a cleaning function of the cleaning and/or treatment device based on the sensing result. This may be a mechanical cleaning function comprised by the oral cleaning and/or treatment device, such as an oscillation action of bristles comprised by the device, or a fluid ejection action in the case of an oral irrigation device or flossing device.

For example, a frequency or amplitude of bristle oscillation may be increased responsive to detecting a tooth or interdental space in the gap. A pulsed ejection of fluid (air or liquid) may be triggered from a nozzle of a flossing or irrigation device in some examples. Certain active agents or substances may be automatically released responsive to detecting presence of an interdental gap or a tooth in some examples.

The response action in each case may be for performing an additional cleaning action. This enables enhanced cleaning to be performed in the interdental space region for example, which is typically difficult to thoroughly clean.

In accordance with one or more embodiments, the first protruding structure and second protruding structure may each comprises at least three electrodes, located at spaced positions along a length of the gap defined between the protruding structures.

This permits sensing at multiple locations along the length of the gap, e.g. between respective co-operative pairs of electrodes at different positions, each pair formed of one of the first electrodes and one of the second electrodes. Thus, three pairs of electrodes across the gap may be provided. This adds spatial definition or resolution to the sensing function. For example, if the middle pair of electrodes senses an interdental space, while the other two do not, this gives an indication that the interdental space is located at a center of the arrangement of three electrodes.

A drive signal may be applied across each pair of electrodes by the RF generator.

The oral cleaning and/or treatment device may further comprise a handle, and/or a body portion, which may be directly or indirectly coupled to the cleaning unit portion. It may be releasably coupleable to the cleaning unit portion.

The oral cleaning and/or treatment device according to some embodiments may be a toothbrush, the cleaning unit being a brush head.

The oral treatment device may in some examples comprise an oscillation mechanism arranged to drive the cleaning unit to oscillate.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an example cleaning unit according to one or more embodiments;
Figs. 2 and 3 illustrates an example cleaning unit in use for cleaning a tooth;
Fig. 4 shows a further example cleaning unit according to one or more embodiments;
Fig. 5 shows an example cleaning unit according to an embodiment which comprises bristles;
Fig. 6 shows a further example cleaning unit according to an embodiment which comprises bristles;
Fig. 7 shows a further example cleaning unit which comprises bristles;
Fig. 8 shows an example cleaning unit in which electrodes are comprised within bristles;
Fig. 9 shows a further example cleaning unit in which electrodes are comprised within bristles;
Fig. 10 shows a further example cleaning unit in which electrodes are comprised within bristles;
Figs. 11 and 12 show an example cleaning unit comprising curved bristles;
Figs. 13 and 14 show a further example cleaning unit comprising curved bristles;
Figs. 15 and 16 show a further example cleaning unit having an H-shaped cross-section;
Fig. 17 shows a further example cleaning unit according to one or more embodiments;
Fig. 18 shows components of an example oral cleaning and/or treatment device;
Fig. 19 shows components of an example oral cleaning and/or treatment device comprising sensing functionality;
Fig. 20 illustrates use of an example cleaning and/or treatment device for sensing of an interproximal space;
Fig. 21 illustrates use of a further example cleaning and/or treatment device having sensing functionality; and
Fig. 22 shows an example toothbrush device according to one or more embodiments.

### DETAILED DESCRIPTION OF THE EMBODIMENTS

The invention will be described with reference to the Figures.

It should be understood that the detailed description and specific examples, while indicating exemplary embodiments of the cleaning unit and device, are intended for purposes of illustration only and are not intended to limit the scope of the invention. These and other features, aspects, and advantages of the cleaning unit and device of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an oral cleaning unit, e.g. a head for an oral cleaning and/or treatment device, which includes electrodes for emitting RF waves for providing an oral cleaning function. The unit includes at least a first and second protruding structure, which protrude in a same general direction away from a base or support structure. Each comprises one or more electrodes. The protruding structures are arranged facing one another and with a gap defined between them which is configured so as to be able to receive a tooth pushed or inserted therein between the first and second protruding structures, and with the protruding structures extending on either side of the tooth, e.g. straddling the tooth. This allows the electrodes comprised by the protruding structures to reach to a location at least partway down side faces of the tooth, closer to interproximal spaces, enabling interproximal spaces to be better cleaned using the RF emissions when the electrodes are suitably driven.

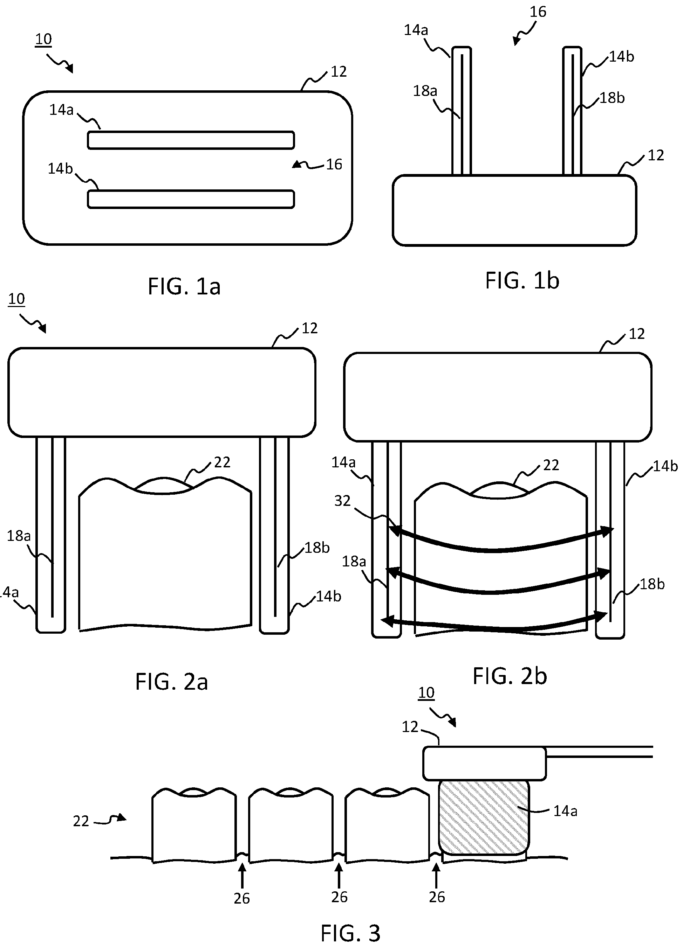

Fig. 1 schematically depicts a simple first example of a cleaning unit for an oral cleaning and/or treatment device in accordance with embodiments of the present invention.

The cleaning unit 10 comprises a first protruding structure 14a, extending outward from a surface of the cleaning unit. The cleaning unit in this example comprises a back-plate element, such a platen 12 from which the protruding structures extend.

The first protruding structure comprises a first electrode, which in this case is integrated within the body of the protruding structure. For example, the protruding structure in this case comprises a laminar protruding member having an electrode integrated therein. **In** other examples, the electrode may be exposed at a surface of the protruding structure or the protruding structure itself may be formed from a conductive material to act as an electrode.

The electrode may take a variety of shapes or structures. It may comprise a flat, planar electrode, e.g. an electrode plate, or may comprise an elongate wire in some examples, or may be shaped in the form of a loop in some examples. A flat planar electrode is the preferred examples.

Part of each of the electrodes 18a, 18b may in some examples be exposed, for example an end portion of each electrode may be exposed at an end of the respective protruding structure.

A second protruding structure 14b is further provided extending outward from the same surface of the cleaning unit 10 and comprising a second electrode 18b.

The second protruding structure 14b is spaced from the first protruding structure 14a by a gap 16.

As shown in Fig. 2, the gap 16 is arranged such that a tooth 22 is receivable in the gap between the first 14a and second 14b protruding structures, with the first and second protruding structures extending either side of the tooth, and wherein, with the tooth received in the gap, the first and second protruding structures extend at least partway down outer side surfaces of the tooth.

The first and second protruding structures 14a, 14b may each comprise more than one electrode 18 in further examples, for example they may each comprise an array of electrodes distributed along the length of the protruding structure.

The gap 16 preferably extends from the first protruding structure 14a to the second protruding structure 14b.

The gap 16 is for receiving the full width of the body of a tooth 22 between the first 14a and second 14b protruding structures.

The electrodes 18a, 18b comprised by the first 14a and second 14b protruding structures are arranged such that, with a tooth 22 received in the gap 16 between the protruding structures, the electrodes are each positioned or extend at least partway down a side surface of the tooth. **In** this way, the electrodes are positioned adjacent the interproximal spaces between the teeth which enables a higher intensity field to reach the interproximal spaces, which enhances cleaning.

The cleaning unit 10 is a cleaning unit for use with an RF-energy based oral cleaning and/or treatment device. For example, each of the first 18a and second 18b electrodes may be suitable for being driven with an RF drive signal to generate an RF electromagnetic field. The cleaning unit is for being received within the oral cavity of a user.

In particular, the electrodes 18a, 18b are for driving with an RF drive signal to generate an RF field within a space proximal and between the electrodes for performing an oral cleaning function when the device is received in an oral cavity of a user.

Each of the first and second electrodes 18a, 18b may be connected to a respective electrical input or terminal (not shown) for receiving an RF driving signal.

As discussed, and as shown in Fig. 2a, the cleaning unit 10 is configured in a form such that a tooth 22 can be fitted in-between the two sets of one or more electrodes 18a, 18b comprised by the protruding structures 14a, 14b, with the electrodes held positioned at least partway down the outer side faces of the tooth. This way the tooth itself acts as the insulating barrier between the electrodes, replacing the need for a dedicated barrier element.

As shown in Fig. 2b, this arrangement also improves cleaning of interproximal spaces because the electrodes 18a, 18b, when the protruding structures 14 are lowered over a tooth 22, are positioned closer to the interproximal spaces, and at the same height level as the spaces. Because the tooth provides an insulating barrier, the RF oscillations (illustrated by arrows 32 in Fig. 2b) are naturally induced to flow or transmit between the electrodes 18a, 18b via the interproximal spaces, i.e. along a pathway which extends through the interproximal spaces at either side of the tooth.

Fig. 3 shows the cleaning unit 10 in use with the protruding structures 14a, 14b, positioned over the tooth 22, so that one protruding structure is on either side of the tooth. Only the first protruding structure 14a is visible in Fig. 3. The interproximal spaces between the teeth are indicated by arrows 26. The cleaning unit 10 in this example is shown attached to a handle or body portion of an example oral cleaning and/or treatment device to which it might be coupled. As illustrated, the side edges of the protruding structure 14a are adjacent the interproximal spaces 22 at either side of the tooth, meaning that the RF energy is induced in use to flow from the electrode(s) 18a in the first structure through the interproximal space 22 to the second structure 18b.

Although in Figs. 1-3, the first and second protruding structures 14a, 14b each comprise a single laminar protruding member which defines an elongate footprint on the cleaning unit surface (i.e. it extends from a line on the cleaning unit surface), other configurations are possible.

According to some examples for instance, each protruding structure may comprise a plurality of protruding members, for example arranged in a respective row, with the row of the first protruding structure facing the row of the second structure. Each protruding member may comprise a respective electrode.

The one or more protruding members of each protruding structure may be flexible. This can be useful for permitting the tooth to be accommodated in the gap between the first and second protruding structures.

For example, the protruding members of the first 14a and second 14b protruding structures may be adapted to flex to receive the tooth 22 in the gap 16. The protruding members may be such that, when the tooth is received in the gap, the members resiliently engage with outer faces of the tooth.

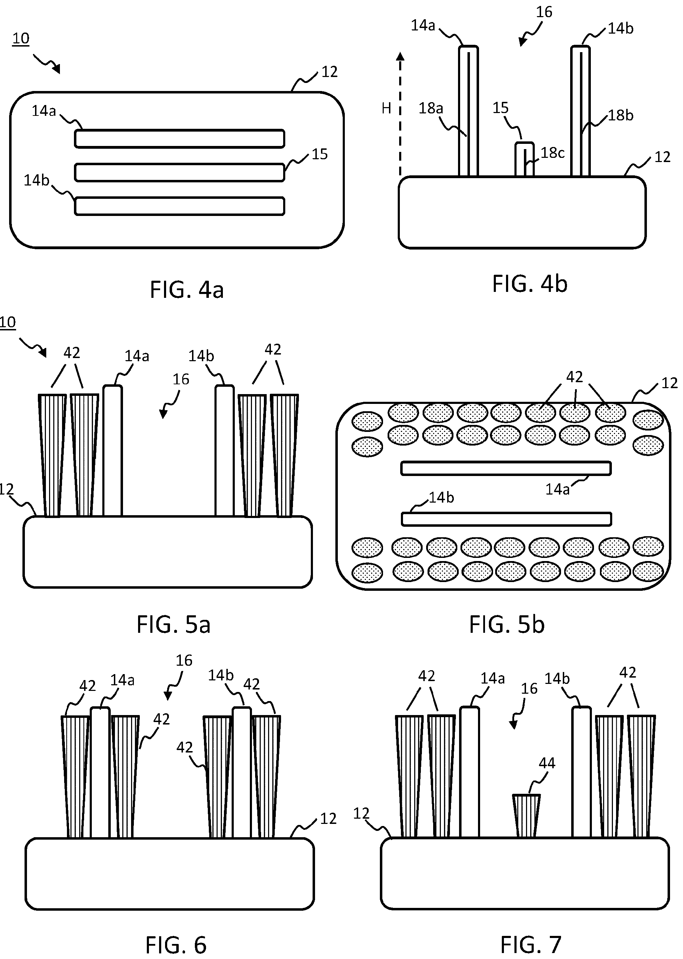

Fig. 4 shows a further example cleaning unit 10 according to one or more embodiments.

In this example, the cleaning unit 10 further comprises a further protruding structure 15 extending outward from a surface of the cleaning unit, the further protruding structure 15 comprising one or more further electrodes 18c, and the further protruding structure disposed in the gap 16 between the first 14a and second 14b protruding structures, and protruding to a shorter height than the first and second protruding structures. The height direction is indicated by arrow H in Fig. 4b.

The further protruding structure 15 comprises a shorter set of one or more electrodes 18c which allow an RF field to be applied in the vicinity of the crown of the tooth 22 when the tooth is received in the gap 16.

There are a number of different options as to how the protruding structures 14a, 14b of the cleaning unit may be implemented according to different embodiments. This may in part depend upon the intended application of the cleaning unit, i.e. the type of oral care device that the cleaning unit will be used for.

According to one set of embodiments, the cleaning unit may comprise a plurality of bristles for cleaning teeth, and wherein the protruding structures 14 are incorporated within or around the bristles of the cleaning unit. This may be suitable for where the cleaning unit is for use with a toothbrush device for example. For example, it may be a head for a toothbrush device.

For example, according to one set of embodiments, the cleaning unit may comprise a platen, and further comprise a plurality of bristles extending outwardly from a first face of the platen for cleaning teeth, and wherein the plurality of bristles are shaped and/or arranged to permit entry of a tooth into said gap. For example, the bristles may be shaped, profiled, or simply positioned in such a way as to not impede receipt of the tooth from above the platen down into the gap between the first and second protruding structures. The configuration of the bristles is so as to permit entry and accommodation of a tooth within said gap. For example, distal ends of the plurality of bristles may define a profile shaped to permit entry and accommodation of a tooth within said gap.

Fig. 5 shows a first example.

In this example, the cleaning unit comprises a platen 12 from which a plurality of bundles of bristles 42 extend in the same direction as the first 14a and second 24b protruding structures. In this example, the bristles are arranged on only one side of the protruding structures, with the gap 16 comprising no bristles. As shown in Fig. 5b, the bundles of bristles 52 are positioned in an arrangement which extends around a peripheral region of the platen 12. Bristles may be provided extending around the whole of the periphery of the platen surface, or, as shown in Fig. 5b, spaces may be left at either end of the gap 16, to permit receipt of teeth into the gap without other neighboring teeth fouling on the bristles, or being impeded by the bristles.

The tooth 22 may be received into the gap 16 in a direction down toward the platen first surface, with the first and second protruding structures 14a, 14b being lowered down over the tooth in question with their distal tips pointing in the direction of movement.

Fig. 6 shows a second example of a cleaning unit 10 comprising bristles 42. In this example, the bristles are arranged surrounding the protruding structures 14a 14b on either side of each of the protruding structures, so that the protruding structures are nestled among the bristles comprised by the cleaning unit. The protruding structures 14a, 14b may be formed of a flexible material to permit flexing of the protruding structures with the bristle bundles 42 as the teeth are brushed by a user using the cleaning unit 10.

Fig. 7 shows a further example. In this example, the cleaning unit 10 comprises an intermediate set of bristles 44, positioned in the gap 16 between the first protruding structure 14a and the second protruding structure 14b. The intermediate set of bristles 44 protrudes to a shorter height than each of the first and second subsets of bristles. The distal ends of the intermediate set of bristles 44 thereby effectively defines a lower boundary to the gap 16 for receiving the tooth 22.

This arrangement means that the intermediate bristles 44 can brush the crown of the tooth 22 received in the gap at the same as RF energy is applied by the electrodes 18 of the first and second protruding structures 14a, 14b.

In accordance with an advantageous set of embodiments, the first 14a and second 14b protruding structures may be at least partially formed by first and second subsets respectively of a plurality of bristles 42 comprised by the cleaning unit 10.

One or more of the bristles of the first subset of bristles may comprise the one or more first electrodes, and one or more bristles of the second subset may comprise the one or more second electrodes.

The first and second subsets of bristles may each form a respective spatial group of bristles which defines the protruding structure.

This can be implemented in different ways. Each of the first electrodes and second electrodes may be themselves provided be in the form of bristles in some examples. For example one or more of the bristles may be formed of a conductive polymer, or other conductive material to enable it to act as an electrode. Alternatively, one or more of the bristles may simply carry or integrate one or more electrodes on or within it.

By way of example, electrodes in the form of bristles may be provided by nylon bristles that are metalized on at least a portion of an outer surface. The conductive metallization functions as the electrode. In some examples, the bristle may be selectively metalized so that it comprises conductive and non-conductive segments.

By way of a further example, electrodes in the form of bristles may be provided by bristles formed of a conductive silicone or rubber material.

By way of a further example, electrodes in the form of bristles may be provided by flexible members which comprise conductive elements (e.g. one or more thin metal wires or plates) embedded or otherwise integrated therein. The flexible member may comprise an insulating sheath which surrounds the inner conductive element. This insulating sheath may be formed for instance of a non-conductive silicone or elastomeric rubbers or thermoplastic polymers suitable for tooth cleaning (e.g. polyamide 6.12, TPE, polybutylene terephthalate).

Fabrication of electrodes in the form of bristles can be achieved by means of standard fabrication techniques such as printed electronics which are overmolded, or by lamination techniques.

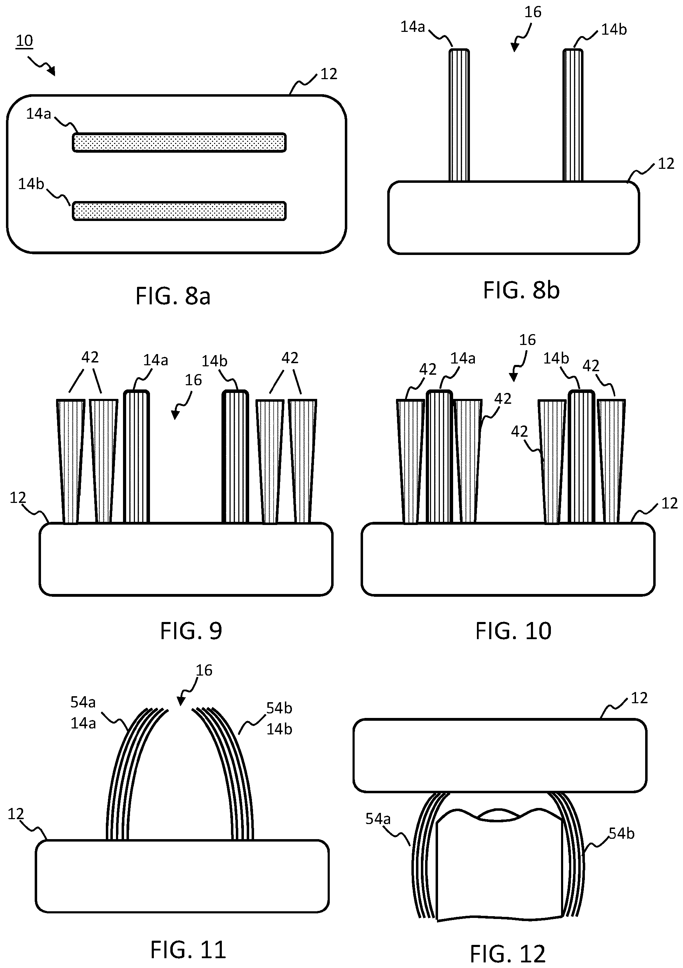

A simple first example of the concept is shown in Fig. 8. Here, each of the first 14a and second 14b protruding structures is formed as a respective spatial group of bristles in the form of a linear bundle of bristles. The bristles of the first protruding structure are arranged in a first linear arrangement and the bristles of the second protruding structure are arranged in a second linear arrangement. The individual bristles form protruding members, the totality of which form the overall protruding structure. The first and second linear arrangements are facing one another, and with a spacing between them defining the gap 16 for receiving a tooth.

In this example, there are no other bristles comprised by the cleaning unit other than the first and second set of bristles forming the first and second protruding structures.

However, in other examples, the bristles forming each of the first and second protruding structures may be simply a subset of the plurality of bristles comprised by the cleaning unit.

Figs. 9 and 10 show two examples of such an arrangement.

In the example of Fig. 9, each of the first and second protruding structures is formed of a respective subset of bristles, the subset of bristles formed as a linear bundle which extends in a line along the surface of the cleaning unit, the lines of the first and second protruding structures facing one another. In the example of Fig. 9, the bristle subsets forming the first and second protruding structures are neighbored by further ordinary bristles of the cleaning unit on only one side of each the protruding structures. In the example of Fig. 10, the subsets of bristles forming the first and second protruding structures are nestled among the remaining plurality of bristles comprised by the cleaning unit, for example surrounded on all sides by the remaining ordinary bristles.

In each of the above examples, the bristles forming each of the protruding structures 14, may each comprise or consist of a respective electrode, or only a subset of the bristles forming each of the protruding structures may comprise or consist of an electrode.

As discussed above, in some examples, the cleaning unit may comprise an intermediate set of bristles, positioned between the first and second subsets of bristles forming the first and second protruding structures 14a, 14b respectively, and the intermediate set of bristles having a shorter length than each of the first and second subsets of bristles. The distal ends of the intermediate set of bristles in this case may define a lower boundary to said gap for receiving the tooth.

The intermediate set of bristles may form a further protruding structure 14, and may comprise one or more further electrodes in some examples.

According to an advantageous set of embodiments, each of the first and second subsets of bristles may comprise a plurality of curved bristles, the curved bristles of each subset arranged to curve inward toward the gap 16 between the first and second protruding structures.

An example of such an embodiment is illustrated in Fig. 11 and Fig. 12. The cleaning unit 10 comprises a first 54a and second 54b bundle of curved bristles forming the first 14a and second 14b protruding structures respectively. The bristles of each of the first and second protruding structures include one or more electrodes, which may be in the form of bristles, or may be comprised by one or more of the bristles in each bundle, e.g. integrated within bristles.

The protruding structures 14a, 14b in this set of embodiments thus effectively define a U-shape gap for receiving the tooth.

As shown in Fig. 12, by providing the electrodes within bristle bundles which curve inward toward the gap 16, when the tooth 22 is received in the gap, the tips of the bristles are biased against side surfaces of the tooth. This both allows the tooth to be brushed by the bristles for a cleaning function, but also ensures that the electrodes among the bristles are held close against the sides of the teeth, maximizing the RF cleaning functionality.

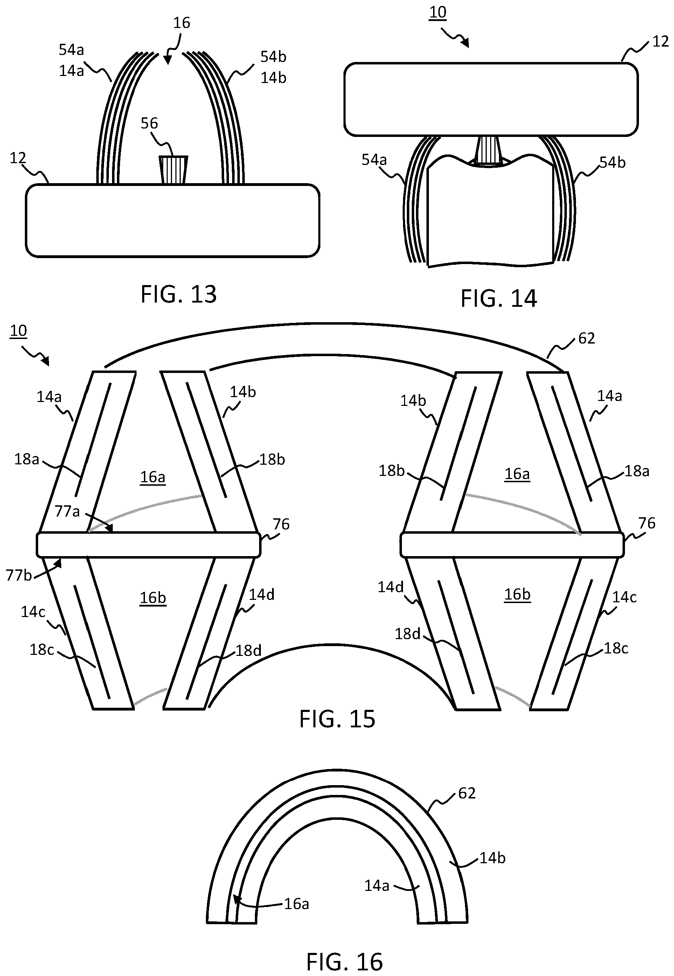

Figs. 13 and 14 illustrate a further example in which the protruding structures 14a, 14b each comprise a respective set of bristles which are curved inward toward the gap 16. In this example, the cleaning unit 10 comprises a further intermediate set of bristles 56 situated in the gap between the protruding structures, similar to the example of Fig. 7 above. This intermediate set of bristles extends to a shorter height that either of the bristle bundles 54a, 54b forming the first and second protruding structures 14a. 14b, and can permit brushing of a crown of the tooth when the tooth is received in the gap, as shown in Fig. 14. The intermediate bristle bundle 56 may form a further protruding structure and comprise one or more further electrodes, to enable the intermediate bristles to perform an RF cleaning function near to the crown of the tooth while the first and second protruding structures 14a, 14b perform cleaning near to the sides of the tooth and the interproximal spaces.

In some examples, the tips of the curved bristles may be arranged at an angle relative to the base of the curved bristles. In particular, each of the plurality of curved bristles may comprise a tip and further comprise a base affixed to the cleaning unit at the platen 12 upper face and wherein the tips of the curved bristles are positioned at an angle relative to the base of the curved bristles.

The plurality of curved bristles may be positioned at an angle of approximately 60 to 120 degrees relative to the base of the curved bristles in some examples.

According to any of the above embodiments, each of the protruding structures may define an elongate footprint on the surface of the cleaning unit from which it protrudes, such that the gap has a length defined by a length of said elongate footprint.

The gap may be elongate.

Above have been discussed a set of embodiments in which the cleaning unit 10 comprises bristles, for providing a dental brushing function.

In other non-limiting examples, the cleaning unit may take a different form. For example, the cleaning unit may take the form of a mouthpiece, or a head for a non-brushing oral treatment device.

Figs. 15 and 16 shows a further example cleaning unit in accordance with one set of embodiments, in which the cleaning unit is in the form of a mouthpiece unit 62. Fig 15 shows a cross-section through the unit, while Fig. 16 shows a plan view of the unit.

In this example, the cleaning unit 10 comprises a support body 76, the support body having a first side 77a facing a first direction, and a second (opposite) side 77b facing in a second direction, opposite to the first direction, and wherein the first 14a and second 14b protruding structures extend outward from the first side 77a, and separated by a first gap 16a.

The cleaning unit 10 further comprises a third protruding structure 14c extending outward from a surface of the second side 77b of the support body 76 and comprising one or more third electrodes 18c, and a fourth protruding structure 14d extending outward from the second side 77b of the support body 76 and comprising one or more fourth electrodes 18d, and the third protruding structure spaced from the fourth protruding structure by a further gap 16b for receiving a tooth therein.

This structure allows teeth from the upper and lower sets of teeth to be both received and cleaned at the same time, with one tooth received in the first gap 16a, on the first (upper) side 77a, and a second tooth received in the second gap 16b beneath the first gap, on the second side 77b, and with the support body 76 providing a spacer or barrier between the received teeth.

In the example shown in Fig. 15, the third 14c and fourth 14d protruding structures are arranged in alignment with the first 14a and second 14b protruding structures respectively, such that the gap 16a and the further gap 16b are in alignment along an axis parallel said first and second directions. However, in further examples, the gap 16a and further gap 16b may be offset from one another in a direction perpendicular said axis to accommodate different upper and lower jaw arcs for example. Offsetting the third protruding structure from the first and the fourth protruding structure from the second may also be beneficial, in spatially separating the electrodes carried by each. This may avoid interference between the RF fields generated by each pair of protruding structures. This is particularly beneficial in embodiments (to be described later) in which sensing is further performed.

In the example shown in Fig. 15, the unit is a mouthpiece unit, wherein the support body 76 extends in a U-shape or horse-shoe shape for extending around the contour of the teeth in the mouth. The protruding structures 14 on each side 77a, 77b of the core may follow and extend continuously around the same curved shape of the core to effectively define an upper channel (formed by the gap 16a between the first 14a and second 14b protruding structures) for receiving the upper teeth and a lower channel (formed by the gap 16b between the third 14c and fourth 14d protruding structures) for receiving the lower teeth.

The protruding structures thus each define an elongate footprint on the cleaning unit surface, so that the gaps have a length commensurate with the length of these elongate footprints.

The cleaning unit 10 may be formed by a single integral or unitary body so that the core 76 and the protruding structures 14 are part of the same integral structure. Thus, the cleaning unit may be formed as a one-piece structure in some examples. This may for example be formed of a flexible material in some examples to permit confirming to the contour of the teeth or mouth.

Although the example of Fig. 15 is in the form of a mouthpiece unit, this is not essential. In other examples, the cleaning unit may take a different form. For example, the support body 76 may be in a laminar or planar form, with respective pairs of protruding structures extending from either side of the planar support body. This could be a head for a cleaning and/or treatment device in some examples, the device having a handle for holding the head within the oral cavity, and the head permitting cleaning of upper and lower teeth at the same time.

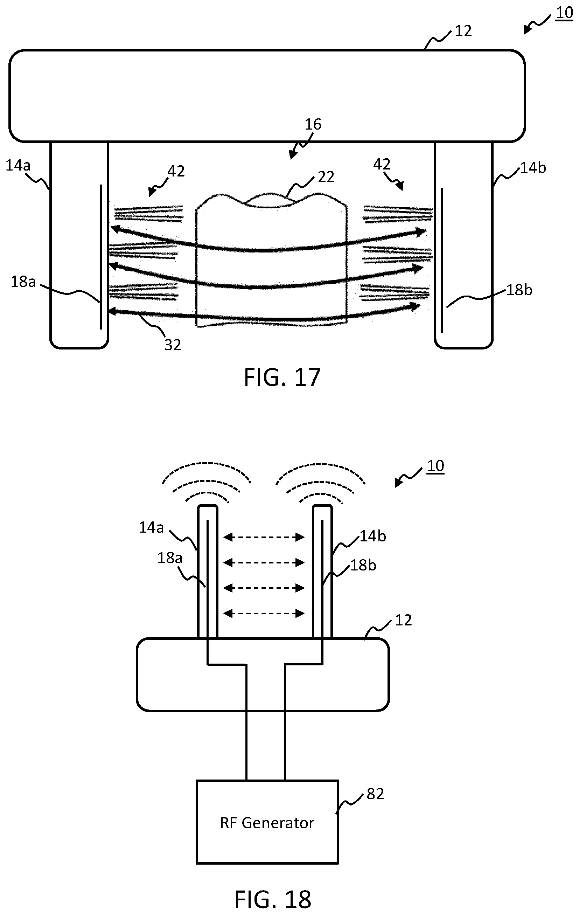

One further example arrangement for an oral cleaning unit 10 according to one or more embodiments is shown in Fig. 17. In this example, the cleaning unit comprises a plurality of bristles 42 which protrude from a surface of each of the first 14a and second 14b protruding structures inward into the gap 16 between the structures. In particular, each of the first and second protruding structures has a side face, facing inward toward the gap, and a plurality of bristles 42 protruding from the side face of the protruding structure into the gap. The bristles of each protruding structure may extend in a direction toward the other, opposing, protruding structure for example.

The bristles 42 extend only partway into the gap 16 between the protruding structures, so that the gap has a free space defined region between distal ends of the opposing sets of bristles 42 within which a tooth 22 can be received. When a tooth is received in the gap 22, as shown in Fig. 17, the bristles may engage with side surfaces of the tooth to provide a physical cleaning function for example. The electrodes 18a, 18b, are integrated in, or carried by, the protruding structures, such that an RF field 32 is generated in the region between the first 18a and second 18b electrodes. In use, this may extend through an interproximal space at a side of the tooth, as shown in Fig. 17.

The structure shown in Fig. 17 may in some cases form of a portion of a cleaning unit such as a cleaning head for a cleaning and/or treatment device, or a portion of a mouthpiece unit for example. For instance, the platen 12 shown in Fig. 12 may form at least part of the support body 76 of a mouthpiece unit, such as that of Fig. 15.

Examples in accordance with a further aspect of the invention provide an oral cleaning and/or treatment device or apparatus.

Components of one example oral cleaning and/or treatment device or apparatus are shown schematically in Fig. 18.

The device or apparatus comprises: a cleaning unit 10 in accordance with any of the examples or embodiments discussed above or described below, or in accordance with any claim of this application. The device further comprises an RF generator 82 in electrical communication with the first electrodes 18a and the second electrodes 18b and configured to drive the electrodes with an alternating potential to induce a radio frequency (RF) electromagnetic field in an area around and between the electrodes, to provide an oral cleaning function when the cleaning unit is received in an oral cavity.

As discussed above, the RF energy interacts with surfaces of teeth and gums to provide a cleaning function.

The RF generator 22 may be electrically coupled to the electrodes 18a, 18b and arranged to supply an RF drive signal to the pair of electrodes to induce generation of an RF field. The alternating drive signal may be applied between the first and second electrodes 18a, 18b so that an alternating potential difference is induced between the electrodes. Hence the first and second electrodes effectively form a pair of electrodes, and this pair may effectively form a capacitor arrangement coupled across an AC supply voltage or AC current from the RF generator.

The RF generator may drive the first and second electrodes with a drive frequency of anywhere between 3 kHz-300 GHz, but more preferably between 500 kHz - 30 MHz for example.

The device may further include a controller or processor for controlling the RF generator. User control means may be provided, e.g. buttons, to allow a user to toggle the RF cleaning on and off.

According to one or more embodiments, the device may further comprise a sensing function for sensing physical bodies or objects brought into the gap between the first and second protruding structures.

The device may be adapted to perform sensing in a region between the first electrodes and the second electrodes, based on sensing variations in one or more electrical characteristics of a circuit comprising the first and second electrodes.

For example, the RF generator may be electrically connected to the first and second electrodes and arranged to apply an alternating drive signal across the electrodes. The circuit comprising the RF generator, and the first and second electrodes forms an RF generation circuit. Sensing may be based on monitoring electrical characteristics of the RF generation circuit.

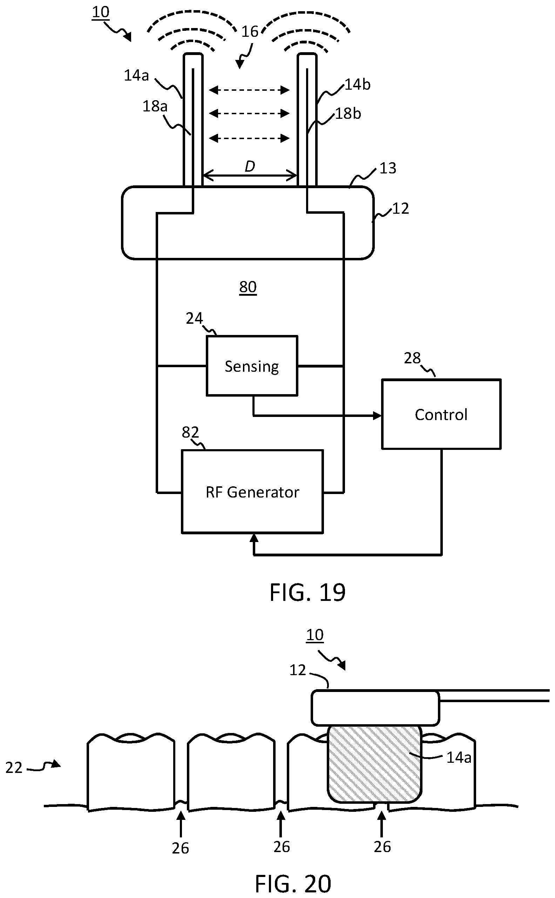

An example circuit arrangement for an oral care device according to one or more embodiments is schematically depicted in Fig. 19.

The device 80 includes an RF generator 22 which is electrically coupled to the electrodes 18a, 18b and arranged to supply an RF drive signal to the pair of electrodes to induce generation of an RF field. The RF field is adapted to perform an oral cleaning function when the cleaning unit is received in an oral cavity.

The alternating drive signal may be applied between the electrodes 18a, 18b so that an alternating potential difference is induced between the electrodes. Hence the pair of electrodes effectively forms a capacitor arrangement coupled across an AC supply voltage from the RF generator.

The device 80 further includes means a sensing unit 24 which is electrically coupled to the pair of electrodes 18a, 18b. The sensing unit 24 is adapted to sense variations in electrical characteristics of the circuit comprising the electrodes.

In the example of Fig. 1, the sensing unit 24 is connected in electrical parallel with the pair of electrodes (and with the RF current generator), meaning it is operable to sense variations in a voltage between the electrodes due to capacitance change induced by variations of electrode distance D or electrical permittivity of the (foreign) material brought in between the electrodes.

In further examples, a sensing unit 24 may be provided in a different electrical arrangement, for example connected in series with one or more of the electrodes 18a, 18b.

The device 80 further comprises a controller 28 which is arranged to receive and process a sensing signal from the sensing unit 24.

In some examples, the controller 28 may be further communicatively or operatively coupled to the RF generator 22 such that the results of the analysis of the sensing signal from the sensing unit 24 can be used for example to inform the RF drive scheme implemented by the RF generator.

In some examples, the controller 28 may be adapted to control the operation of the RF generator 22.

The circuit comprising the electrodes 18a, 18b, and the RF generator 82 can be understood as together forming an RF generation circuit. In the example of Fig. 19, the sensing unit 24 is arranged electrically connected to this circuit, such that sensing is based on detecting variation in one or more electrical characteristics of the RF generation circuit.

The controller 28 may be adapted to detect presence of a dental feature or structure in a region between the first 14a and second 14b protruding structures (e.g. in the gap 16 between them), based on sensing variations in one or more electrical characteristics of the circuit comprising the first and second electrodes.

In advantageous embodiments, the controller 28 may be adapted to sense presence of a tooth 22 received in the gap 16 between the first and second protruding structure, or sense presence of an interdental space 26 within the gap.

The sensing may be based on sensing variation in a voltage across the first 18a and second 18b electrodes.

The sensing may be based on directly or indirectly sensing a change in capacitance between the first 18a and second 18b electrodes.

For instance, for a first 14b and second 14b protruding structure separated by a gap of distance, D, if the protruding structures flex outward from the gap slightly as a tooth is received in the gap, the distance, D, changes (increases) and the capacitance between the electrodes 18a, 18b changes accordingly. The change in capacitance, or impedance, may be measured based on sensing a voltage change over the electrode pair (formed of the first electrode(s) and second electrode(s)).

Likewise, if one or both of the protruding structures is received or inserted in an interdental space, this may also alter the gap distance D between the first and second electrodes, leading to a detectable change in capacitance.

Furthermore, if a tooth is received in the gap, the presence of this object in the space between the electrodes may change the overall electric permittivity of the space between the electrodes which may change the effective capacitance between them in a detectable way.

As mentioned, the controller 28 is coupled to the sensing unit 24 and arranged to receive a sensor signal output from the sensor unit, and adapted to detect presence of a dental feature or structure in the gap between the protruding structures based on the sensor signal output.

Particular dental features or structures may be associated with different alterations in the electrical characteristics of the circuit between the electrodes. For example, an open space may be associated with a first effective capacitance between the electrodes (reflected in a measurable voltage across the electrodes), an interdental space between the electrodes associated with a second effective capacitance, and presence of the full width of a tooth in the gap associated with a different effective capacitance. A size of the interdental space may be detectable by the controller based on the detected effective capacitance between the electrodes or other electrical characteristic of the circuit comprising the electrodes.

**In** some examples, the device may include a memory, communicatively coupled to the controller, the memory storing a reference dataset, e.g. a lookup table, recording reference circuit characteristics for each of a plurality of different possible dental features or entities.

The controller 28 may be adapted to trigger one or more response actions responsive to sensing a particular dental feature or structure, such as an interproximal space, in the region between the protruding structures.

For example, an intensity or power level of the RF field may be increased (e.g. by increasing an amplitude or frequency of the RF drive signal, responsive to detecting a tooth or interdental space received in the gap.

The controller may be arranged to adjust a cleaning function of the cleaning and/or treatment device 80 based on the sensing result. This may be a mechanical cleaning function comprised by the oral cleaning and/or treatment device, such as an oscillation action of bristles comprised by the device, or a fluid ejection action in the case of an oral irrigation device or flossing device.

For example, the device may include a mechanical oscillation mechanism arranged to apply an oscillation to the platen 12 to oscillate the bristles, and wherein a frequency or amplitude of bristle oscillation may be increased or decreased responsive to detecting a tooth or interproximal space in the gap. A pulsed ejection of fluid (air or liquid) may be triggered from a nozzle of a flossing or irrigation device in some examples. Certain active agents or substances may be automatically released responsive to detecting presence of an interdental gap or a tooth in some examples.

The response action in each case may be for performing an additional cleaning and/or treatment action. This enables enhanced cleaning to be performed in the interdental space region for example, which is typically difficult to thoroughly clean.

Fig. 20 illustrates use of an example cleaning and/or treatment device. The cleaning head 10 has been positioned over a row of teeth, so that two neighboring teeth 22 are partially received into the gap 16 (not visible) between the first 14a and second 14b protruding structures. The interproximal space 26 between the two neighboring teeth 22 is thus located in the gap between the protruding structures. The controller 28 (not shown) may detect presence of the interproximal gap based on variation in voltage of the RF generation circuit, or change in effective capacitance between the electrodes.

Alternatively, the cleaning head might be rotated 90 degrees and the protruding structures 14a, 14b each received in interproximal spaces either side of one of the teeth 22. In this case, a tooth is fully received in the gap 16 between the protruding structures. The deflection of the protruding structures as they enter the interproximal spaces may be detectable.

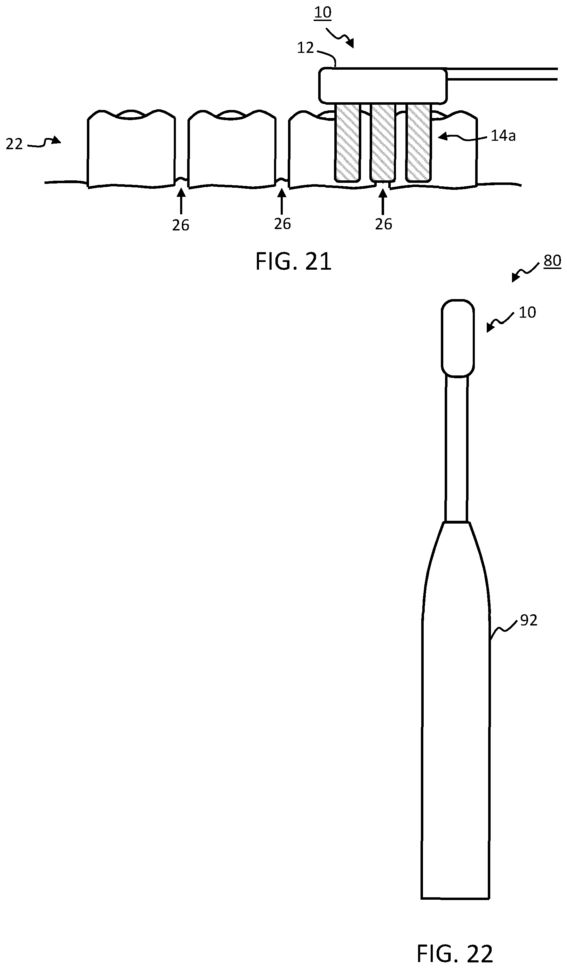

In accordance with one or more embodiments, the first protruding structure and second protruding structure may each comprises at least three electrodes, located at spaced positions along a length of the gap defined between the protruding structures. An example is shown in Fig. 21. Here, each of the first and second protruding structures 14a, 14b is comprised of a plurality of spatially separated portions (three in this example), each comprising a respective electrode.

This permits sensing at multiple locations along the length of the gap 16 between the first and second protruding structures, e.g. between respective co-operative pairs of electrodes at different positions, each pair formed of one of the first electrodes 18a and one of the second electrodes 18b. Thus, three pairs of electrodes across the gap may be provided. This adds spatial definition or resolution to the sensing function. For example, if the middle pair of electrodes senses an interdental gap, while the other two do not, this gives an indication that the interdental gap 26 is located at a center of the arrangement of three electrodes.

A drive signal is applied across each pair of electrodes by the RF generator 82 for example.

As mentioned above, results of the sensing may be communicated to an external device by means of a wired or wireless communication interface in some examples. For instance, the results of the sensing may be uploaded to a server, or may be communicated to a mobile communication device such as a smartphone, e.g. via a wireless communication protocol such as Bluetooth or Wi-Fi.

Results may be monitored over time for clinical monitoring of a subject. For example, increasing size of an interdental space can be detected, or recessing gums, or movement of teeth.

The oral cleaning and/or treatment device may further comprise a handle, and/or a body portion, which may be directly or indirectly coupled to the cleaning unit. The handle or body portion may house or accommodate the RF generator 82, and facilitate electrical connection between the generator and the electrodes of the cleaning unit.

The cleaning unit may be a disposable attachment to a body portion of the oral cleaning and/or treatment device (e.g. a head for a toothbrush device). It may be adapted to releasably mechanically and electrically couple to the body portion.

The oral cleaning and/or treatment device according to some embodiments may be a toothbrush 80, wherein the cleaning unit 10 is a brush head for the toothbrush. One example is shown schematically in Fig. 22, which shows the cleaning unit 10 in the form of a toothbrush head mounted to the distal end of a body portion 92 which houses the RF generator (not shown). The body portion forms a handle for the device.

The oral cleaning device may in some examples comprise an oscillation mechanism arranged to drive the cleaning unit to oscillate, e.g. to oscillate bristles.

Optionally, the cleaning unit 10 may be arranged to be releasably coupleable to the body portion 92, so that the cleaning unit is detachable from the body portion.

A wide variety of different types of oral cleaning device may be provided in accordance with embodiments of the invention.

By way of non-limiting example, the oral cleaning or treatment device according to embodiments the present invention may be any of the following:
- a toothbrush device (e.g. a powered toothbrush);
- an oral irrigator device;
- a powered flossing device;
- a combined brushing and flossing device; or
- a mouthpiece device, e.g. brushing mouthpiece device.

As discussed above, embodiments make use of a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A cleaning unit (10) for an RF-energy based oral cleaning and/or treatment device, the cleaning unit for being received in an oral cavity of a user, the cleaning unit comprising:
a first protruding structure (14a), extending outward from a surface of the cleaning unit, the first protruding structure comprising one or more first electrodes (18a),
a second protruding structure (14b) extending outward from the surface of the cleaning unit, and comprising one or more second electrodes (18),
wherein the one or more first electrodes and one or more second electrodes are suitable for being driven with an RF drive signal to generate an RF electromagnetic field;
the second protruding structure spaced from the first protruding structure by a gap (16), wherein the gap consists of a space extending from the first protruding structure to the second protruding structure,
wherein the gap (16) is arranged such that at least one tooth (22) is receivable in the gap between the first and second protruding structures, with the first and second protruding structures extending on either side of the gap, wherein, with the at least one tooth received in the gap, the first and second protruding structures extend at least partway down outer side surfaces of the tooth,
wherein the first (14a) and second (14b) protruding structures each comprise a plurality of protruding members, and wherein at least a portion of the protruding members of each protruding structure are flexible;
wherein the gap (16) is of a size suitable for receiving the full width of an average molar tooth of an average adult human.

2. A cleaning unit (10) as claimed in claim 1, wherein at least a portion of the protruding members of the first and second protruding structures are adapted to flex to receive the tooth in the gap, such that when the tooth is received in the gap the members resiliently engage with outer faces of the tooth.

3. A cleaning unit (10) as claimed in any of claims 1-2, wherein the cleaning unit comprises a platen (12), and further comprises a plurality of bristles (42) extending outwardly from a first face of the platen for cleaning teeth, and wherein the plurality of bristles are shaped and/or arranged to permit entry of a tooth into said gap (16).

4. A cleaning unit (10) as claimed in claim 3, wherein the first (14a) and second (14b) protruding structures are at least partially formed by first and second subsets respectively of said plurality of bristles (42), the bristles forming the protruding members.

5. A cleaning unit (10) as claimed in claim 4, wherein one or more of the bristles of the first subset of bristles comprise the one or more first electrodes (18a), and one or more bristles of the second subset comprise the one or more second electrodes (18b).

6. A cleaning unit (10) as claimed in claim 4 or 5, wherein the first subset of bristles is arranged in a first spatial group and the second subset of bristles is arranged in a second spatial group, the first and second groups having a spacing between them defining said gap (16).

7. A cleaning unit (10) as claimed in any of claims 3-6, wherein the first (18a) and second electrodes (18b) are in the form of bristles.

8. A cleaning unit (10) as claimed in any of claims 4-7, wherein the cleaning unit comprises an intermediate set of bristles (44, 56), positioned between the first subset of bristles and the second subset of bristles, and the intermediate set of bristles having a shorter length than each of the first and second sets of bristles, and wherein distal ends of the intermediate set of bristles define a lower boundary to said gap for receiving the tooth.

9. A cleaning unit (10) as claimed in any of claims 4-8, wherein each of the first and second subsets of bristles comprise a plurality of curved bristles (54a, 54b), the curved bristles of each subset arranged to curve in a direction inward toward the gap (16).

10. A cleaning unit (10) as claimed in any of claims 1-9,
wherein the cleaning unit comprises a support body (76), the support body having a first side (77a) facing a first direction, and a second side (77b) facing in a second direction, opposite to the first direction, and wherein the first (14a) and second (14b) protruding structures extend outward from the first side (77a), and
the cleaning unit further comprising a third protruding structure (14c) extending outward from a surface of the second side (77b) of the support body (76) and comprising one or more third electrodes (18c), and a fourth protruding (14d) structure extending outward from the second side (77b) of the support body and comprising one or more fourth electrodes (18d), and the third protruding structure spaced from the fourth protruding structure by a further gap (16b) for receiving a tooth (22) therein.

11. A cleaning unit (10) as claimed in claim 10, wherein the third (14c) and fourth (14d) protruding structures are arranged in alignment with the first (14a) and second (14b) protruding structures respectively, such that the gap (16a) and the further gap (16b) are in alignment along an axis parallel said first and second directions.

12. An RF-energy based oral cleaning and/or treatment device comprising:
a cleaning unit (10) as claimed as in any of claims 1-11, and
an RF generator (82) in electrical communication with the first electrodes (18a) and the second electrodes (18b) and configured to drive the electrodes with an RF alternating drive signal to induce an RF alternating field in an area around and between the electrodes, to provide an oral cleaning function when the cleaning unit is received in an oral cavity.

13. An RF-energy based oral cleaning and/or treatment device as claimed in claim 12, wherein the device comprises a controller adapted to detect presence of a dental feature or structure in a region between the first and second protruding structures, based on sensing variations in one or more electrical characteristics of a circuit comprising the first and second electrodes, and
optionally wherein the controller is adapted to detect presence of a tooth received in the gap (16) between the first (14a) and second (14b) protruding structures, or presence of an interdental space in the gap.

## Patentansprüche

1. Reinigungseinheit (10) für eine auf RF-Energie basierende orale Reinigungs- und/oder Behandlungsvorrichtung, wobei die Reinigungseinheit dazu bestimmt ist, in einer oralen Kavität eines Benutzers empfangen zu werden, wobei die Reinigungseinheit Folgendes umfasst:
eine erste vorstehende Struktur (14a), die sich von einer Oberfläche der Reinigungseinheit nach außen erstreckt, wobei die erste vorstehende Struktur eine oder mehrere erste Elektroden (18a) umfasst,
eine zweite vorstehende Struktur (14b), die sich von der Oberfläche der Reinigungseinheit nach außen erstreckt und eine oder mehrere zweite Elektroden (18) umfasst,
wobei die eine oder mehreren ersten Elektroden und die eine oder mehreren zweiten Elektroden geeignet sind, mit einem RF-Ansteuerungssignal angesteuert zu werden, um ein elektromagnetisches RF-Feld zu erzeugen;
die zweite vorstehende Struktur von der ersten vorstehenden Struktur durch einen Spalt (16) beabstandet ist, wobei der Spalt aus einem Raum besteht, der sich von der ersten vorstehenden Struktur zu der zweiten vorstehenden Struktur erstreckt,
wobei der Spalt (16) so angeordnet ist, dass mindestens ein Zahn (22) in dem Spalt zwischen der ersten und der zweiten vorstehenden Struktur aufgenommen werden kann, wobei sich die erste und die zweite vorstehende Struktur auf beiden Seiten des Spalts erstrecken, wobei, wenn der mindestens eine Zahn in dem Spalt empfangen wird, sich die erste und die zweite vorstehende Struktur mindestens teilweise herunter entlang der äußeren Oberflächen des Zahns erstrecken,
wobei die ersten (14a) und zweiten (14b) vorstehenden Strukturen jeweils eine Vielzahl von vorstehenden Gliedern umfassen, und wobei mindestens ein Abschnitt der vorstehenden Glieder jeder vorstehenden Struktur flexibel ist;
wobei der Spalt (16) von einer Größe ist, die geeignet ist, um die volle Breite eines durchschnittlichen Backenzahns eines durchschnittlichen erwachsenen Menschen zu empfangen.

2. Reinigungseinheit (10) nach Anspruch 1, wobei mindestens ein Abschnitt der vorstehenden Glieder der ersten und zweiten vorstehenden Strukturen adaptiert ist, um sich zu biegen, um den Zahn in dem Spalt zu empfangen, so dass, wenn der Zahn in dem Spalt empfangen wird, die Glieder elastisch mit den äußeren Flächen des Zahns in Eingriff kommen.

3. Reinigungseinheit (10) nach einem der Ansprüche 1-2, wobei die Reinigungseinheit eine Platte (12) umfasst und weiter eine Vielzahl von Borsten (42) umfasst, die sich von einer ersten Fläche der Platte nach außen erstrecken, um Zähne zu reinigen, und wobei die Vielzahl von Borsten so geformt und/oder angeordnet ist, dass sie den Eintritt eines Zahns in den Spalt (16) gestatten.

4. Reinigungseinheit (10) nach Anspruch 3, wobei die ersten (14a) und zweiten (14b) vorstehenden Strukturen mindestens teilweise durch entsprechende erste und zweite Teilemengen der Vielzahl von Borsten (42) gebildet werden, wobei die Borsten die vorstehenden Glieder bilden.

5. Reinigungseinheit (10) nach Anspruch 4, wobei eine oder mehrere der Borsten des ersten Teilsatzes von Borsten die eine oder mehreren ersten Elektroden (18a) umfassen und eine oder mehrere Borsten des zweiten Teilsatzes die eine oder mehreren zweiten Elektroden (18b) umfassen.

6. Reinigungseinheit (10) nach Anspruch 4 oder 5, wobei der erste Teilsatz von Borsten in einer ersten räumlichen Gruppe angeordnet ist und der zweite Teilsatz von Borsten in einer zweiten räumlichen Gruppe angeordnet ist, wobei die erste und die zweite Gruppe einen Abstand zwischen ihnen aufweisen, der den Spalt (16) definiert.

7. Reinigungseinheit (10) nach einem der Ansprüche 3-6, wobei die erste (18a) und die zweite Elektrode (18b) in der Form von Borsten sind.

8. Reinigungseinheit (10) nach einem der Ansprüche 4-7, wobei die Reinigungseinheit einen Zwischensatz von Borsten (44, 56) umfasst, der zwischen dem ersten Teilsatz von Borsten und dem zweiten Teilsatz von Borsten positioniert ist, und wobei der Zwischensatz von Borsten eine kürzere Länge als jeder der ersten und zweiten Sätze von Borsten aufweist, und wobei distale Enden des Zwischensatzes von Borsten eine untere Grenze zu dem Spalt zum Empfangen des Zahns definieren.

9. Reinigungseinheit (10) nach einem der Ansprüche 4-8, wobei jeder der ersten und zweiten Teilsätze von Borsten eine Vielzahl von gekrümmten Borsten (54a, 54b) umfasst, wobei die gekrümmten Borsten jedes Teilsatzes angeordnet sind, um sich in eine Richtung des Spalts (16) nach innen zu krümmen.

10. Reinigungseinheit (10) nach einem der Ansprüche 1-9,
wobei die Reinigungseinheit einen Stützkörper (76) umfasst, wobei der Stützkörper eine erste Seite (77a) aufweist, die in eine erste Richtung weist, und eine zweite Seite (77b), die in eine zweite Richtung, der ersten Richtung entgegengesetzt, weist, und wobei die erste (14a) und die zweite (14b) vorstehende Struktur sich von der ersten Seite (77a) nach außen erstrecken, und
die Reinigungseinheit weiter eine dritte vorstehende Struktur (14c), die sich von einer Oberfläche der zweiten Seite (77b) des Stützkörpers (76) nach außen erstreckt umfasst, und eine oder mehrere dritte Elektroden (18c) umfasst, und eine vierte vorstehende Struktur (14d), die sich von der zweiten Seite (77b) des Stützkörpers nach außen erstreckt, und eine oder mehrere vierte Elektroden (18d) umfasst, und wobei die dritte vorstehende Struktur von der vierten vorstehenden Struktur durch einen weiteren Spalt (16b) beabstandet ist, um einen Zahn (22) darin zu empfangen.

11. Reinigungseinheit (10) nach Anspruch 10, wobei die dritte (14c) und vierte (14d) vorstehende Struktur in Ausrichtung mit der entsprechenden ersten (14a) und zweiten (14b) vorstehenden Struktur angeordnet sind, so dass der Spalt (16a) und der weitere Spalt (16b) entlang einer Achse parallel zu der ersten und zweiten Richtung ausgerichtet sind.

12. Auf RF-Energie basierende orale Reinigungs- und/oder Behandlungsvorrichtung, umfassend:
eine Reinigungseinheit (10) nach einem der Ansprüche 1-11, und
einen RF-Generator (82), in elektrischer Kommunikation mit den ersten Elektroden (18a) und den zweiten Elektroden (18b), und konfiguriert, um die Elektroden mit einem alternierenden RF-Ansteuerungssignal anzusteuern, um ein alternierendes RF-Feld in einem Bereich um und zwischen den Elektroden zu induzieren, um eine orale Reinigungsfunktion bereitzustellen, wenn die Reinigungseinheit in einer oralen Kavität empfangen wird.

13. Auf RF-Energie basierende orale Reinigungs- und/oder Behandlungsvorrichtung nach Anspruch 12, wobei die Vorrichtung eine Steuereinheit umfasst, die adaptiert ist, um das Vorhandensein eines Dentalmerkmals oder einer Struktur in einem Gebiet zwischen den ersten und zweiten vorstehenden Strukturen zu erfassen, basierend auf der Erfassung von Variationen in einer oder mehreren elektrischen Eigenschaften eines Schaltkreises, der die ersten und zweiten Elektroden umfasst, und
wobei die Steuereinheit optional adaptiert ist, um das Vorhandensein eines in dem Spalt (16) zwischen der ersten (14a) und der zweiten (14b) vorstehenden Struktur empfangenen Zahns oder das Vorhandensein eines Interdentalraums in dem Spalt zu detektieren.

## Revendications

1. Unité de nettoyage (10) pour un dispositif de nettoyage et/ou de traitement buccal à base d'énergie RF, l'unité de nettoyage étant destinée à être reçue dans une cavité buccale d'un utilisateur, l'unité de nettoyage comprenant :
une première structure en saillie (14a), s'étendant vers l'extérieur à partir d'une surface de l'unité de nettoyage, la première structure en saillie comprenant une ou plusieurs premières électrodes (18a),
une deuxième structure en saillie (14b) s'étendant vers l'extérieur à partir de la surface de l'unité de nettoyage et comprenant une ou plusieurs secondes électrodes (18),
dans lequel les une ou plusieurs premières électrodes et une ou plusieurs secondes électrodes sont adaptées pour être entraînées par un signal d'entraînement RF pour générer un champ électromagnétique RF ;
la deuxième structure en saillie étant espacée de la première structure en saillie par un écart (16), dans laquelle l'écart est constitué d'un espace s'étendant de la première structure en saillie à la deuxième structure en saillie,
dans lequel l'écart (16) est agencé de telle sorte qu'au moins une dent (22) peut être reçue dans l'écart entre les première et seconde structures en saillie, les première et seconde structures en saillie s'étendant de chaque côté de l'écart, dans lequel, avec la au moins une dent reçue dans l'écart, les première et seconde structures en saillie s'étendent au moins partiellement vers le bas de surfaces latérales extérieures de la dent,
dans lequel les première (14a) et deuxième (14b) structures en saillie comprennent chacune une pluralité d'éléments en saillie, et dans lequel au moins une partie des éléments en saillie de chaque structure en saillie sont flexibles ;
dans lequel l'écart (16) est d'une taille compatible pour recevoir la pleine largeur totale d'une molaire d'un adulte humain moyen.

2. Unité de nettoyage (10) selon la revendication 1, dans laquelle au moins une partie des éléments en saillie des première et seconde structures en saillie sont adaptés pour fléchir afin de recevoir la dent dans l'écart, de telle sorte que lorsque la dent est reçue dans l'écart, les éléments viennent en prise de manière résiliente avec des faces extérieures de la dent.

3. Unité de nettoyage (10) selon l'une quelconque des revendications 1-2, dans laquelle l'unité de nettoyage comprend un plateau (12) et comprend en outre une pluralité de poils (42) s'étendant vers l'extérieur à partir d'une première face du plateau pour nettoyer des dents, et dans laquelle la pluralité de poils sont mis en forme et/ou agencés pour permettre l'entrée d'une dent dans ledit écart(16).

4. Unité de nettoyage (10) selon la revendication 3, dans laquelle les première (14a) et deuxième (14b) structures en saillie sont au moins partiellement formées respectivement par des premier et second sous-ensembles de ladite pluralité de poils (42), les poils formant les éléments en saillie.

5. Unité de nettoyage (10) selon la revendication 4, dans laquelle un ou plusieurs des poils du premier sous-ensemble de poils comprennent les une ou plusieurs premières électrodes (18a), et un ou plusieurs poils du second sous-ensemble comprennent les une ou plusieurs secondes électrodes (18b).

6. Unité de nettoyage (10) selon la revendication 4 ou 5, dans laquelle le premier sous-ensemble de poils est agencé dans un premier groupe spatial et le second sous-ensemble de poils est agencé dans un second groupe spatial, les premier et second groupes présentant un espacement entre eux définissant ledit écart(16).

7. Unité de nettoyage (10) selon l'une quelconque des revendications 3-6, dans laquelle les premières (18a) et secondes électrodes (18b) se présentent sous la forme de poils.

8. Unité de nettoyage (10) selon l'une quelconque des revendications 4-7, dans laquelle l'unité de nettoyage comprend un ensemble intermédiaire de poils (44, 56), positionné entre le premier sous-ensemble de poils et le second sous-ensemble de poils, et l'ensemble intermédiaire de poils présentant une longueur plus courte que chacun des premier et second ensembles de poils, et dans laquelle des extrémités distales de l'ensemble intermédiaire de poils définissent une limite inférieure audit écart pour recevoir la dent.

9. Unité de nettoyage (10) selon l'une quelconque des revendications 4-8, dans laquelle chacun des premier et second sous-ensembles de poils comprend une pluralité de poils courbés (54a, 54b), les poils courbés de chaque sous-ensemble étant agencés pour se courber dans une direction vers l'intérieur en direction de l'écart (16).

10. Unité de nettoyage (10) selon l'une quelconque des revendications 1-9,
dans laquelle l'unité de nettoyage comprend un corps de support (76), le corps de support présentant un premier côté (77a) faisant face à une première direction, et un second côté (77b) faisant face à une seconde direction, opposée à la première direction, et dans laquelle les première (14a) et deuxième (14b) structures en saillie s'étendent vers l'extérieur à partir du premier côté (77a), et
l'unité de nettoyage comprenant en outre une troisième structure en saillie (14c) s'étendant vers l'extérieur à partir d'une surface du second côté (77b) du corps de support (76) et comprenant une ou plusieurs troisièmes électrodes (18c), et une quatrième structure en saillie (14d) s'étendant vers l'extérieur à partir du second côté (77b) du corps de support et comprenant une ou plusieurs quatrièmes électrodes (18d), et la troisième structure en saillie étant espacée de la quatrième structure en saillie par un écart supplémentaire (16b) pour recevoir une dent (22) en son sein.

11. Unité de nettoyage (10) selon la revendication 10, dans laquelle les troisième (14c) et quatrième (14d) structures en saillie sont agencées en alignement avec les première (14a) et deuxième (14b) structures en saillie, respectivement, de telle sorte que l'écart (16a) et l'écart supplémentaire (16b) sont alignés le long d'un axe parallèle auxdites première et seconde directions.

12. Dispositif de nettoyage et/ou de traitement buccal à base d'énergie RF, comprenant :
une unité de nettoyage (10) selon l'une quelconque des revendications 1-11, et
un générateur RF (82) en communication électrique avec les premières électrodes (18a) et les secondes électrodes (18b) et configuré pour entraîner les électrodes avec un signal d'entraînement alternatif RF afin d'induire un champ alternatif RF dans une zone autour et entre les électrodes pour fournir une fonction de nettoyage buccal lorsque l'unité de nettoyage est reçue dans une cavité buccale.

13. Dispositif de nettoyage et/ou de traitement buccal à base d'énergie RF selon la revendication 12, dans lequel le dispositif comprend un dispositif de commande conçu pour détecter la présence d'une caractéristique ou d'une structure dentaire dans une région située entre les première et deuxième structures en saillie, en fonction de la détection de variations dans une ou plusieurs caractéristiques électriques d'un circuit comprenant les premières et secondes électrodes.
facultativement dans lequel le dispositif de commande est adapté pour détecter la présence d'une dent reçue dans l'écart (16) entre les première (14a) et deuxième (14b) structures en saillie, ou la présence d'un espace interdentaire dans l'écart.
